(19) Europäisches Patentamt
European Patent Office
Office européen des brevets

(11) Publication number : **0 441 014 B1**

(12) **EUROPEAN PATENT SPECIFICATION**

(45) Date of publication of patent specification : **07.04.93 Bulletin 93/14**

(51) Int. Cl.$^5$ : **C10L 1/22,** C10L 1/14, C07C 51/567, C08F 8/32

(21) Application number : **90301232.6**

(22) Date of filing : **06.02.90**

# (54) Compositions for control of induction system deposits.

(43) Date of publication of application : **14.08.91 Bulletin 91/33**

(45) Publication of the grant of the patent : **07.04.93 Bulletin 93/14**

(84) Designated Contracting States : **BE DE ES FR GB IT**

(56) References cited :
**EP-A- 0 031 236**
**EP-A- 0 237 356**
**WO-A-85/03504**
**DE-A- 2 008 846**
**DE-B- 1 274 266**
**FR-A- 2 201 309**
**FR-A- 2 203 799**
**GB-A- 1 310 847**
**US-A- 3 307 928**

(56) References cited :
**US-A- 3 909 215**
**US-A- 4 234 435**
**US-A- 4 321 062**
**US-A- 4 643 737**

(73) Proprietor : **ETHYL PETROLEUM ADDITIVES LIMITED**
**London Road**
**Bracknell, Berkshire RG12 2UW (GB)**

(72) Inventor : **Wallace, Graeme McRobert**
**5 Freesia Close, Wokingham,**
**Bershire, RG11 2NE, (GB)**
Inventor : **Simmonds, James Patrick,**
**7 Stannard Well Lane, Horbury,**
**Wakefield, West Yorkshire, WF4 6BW (GB)**

(74) Representative : **Collier, Jeremy Austin Grey et al**
**J.A.Kemp & Co. 14, South Square Gray's Inn**
**London WC1R 5EU (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 441 014 B1

Jouve, 18, rue Saint-Denis, 75001 PARIS

## Description

This invention relates to reducing fuel induction system deposits in internal combustion engines. More particularly this invention relates to detergent/dispersant compositions and to distillate fuels and distillate fuel additive concentrates capable of reducing the amount of intake valve deposits formed during engine operation.

A problem frequently encountered in the operation of gasoline and diesel engines is the formation of undesirable amounts of induction system deposits, such as intake valve deposits. An object of this invention is to provide compositions capable of reducing the severity of this problem.

A further problem encountered is the ability of the fuel to shed (i.e. not to emulsify) water which may find its way into the fuel, for example due to condensation. A further object of this invention is to provide an additive system which not only reduces the severity of the deposit problem, but also provides a fuel with satisfactory demulse performance.

This invention provides and utilizes a detergent/dispersant prepared by a process which comprises reacting (i) triethylene tetramine or a combination of ethylene polyamines which approximates triethylene tetramine with (ii) at least one acyclic hydrocarbyl substituted succinic acylating agent characterized in that the substituent contains an average of 50 to 90 (preferably 64 to 80) carbon atoms, and in that such acylating agent has an acid number in the range of 0.9 to 1.3 (preferably in the range of 1.0 to 1.2), said detergent/dispersant containing in its molecular structure in chemically combined form an average of from 1.5 to 2.2 (preferably from 1.9 to 2.1, and most preferably 2.0) moles of (ii) per mole of (i).

In one of its forms, this invention provides a distillate fuel (preferably a gasoline-type fuel) containing a minor, but effective amount, of (a) a detergent/dispersant of the type just described, and optionally, but preferably, (b) an antioxidant, or (c) a demulsifier, or (d) a diluent oil, or (e) a corrosion inhibitor, or any combination of any two or any three or all four of components (b), (c), (d) and (e). Other performance additives such as antifoam agents, combustion improvers, cetane improvers, and octane improvers, may also be present.

Another embodiment of this invention is an additive concentrate which comprises, in combination, (a) a detergent/dispersant of the type just described, and (b) an antioxidant, or (c) a demulsifier, or (d) a diluent oil, or (e) a corrosion inhibitor, or any combination of any two or any three or all four of components (b), (c), (d) and (e).

These and other embodiments of this invention will be apparent from the ensuing description and appended claims.

Detergent/dispersant.

As noted above, the detergent/dispersants of this invention are particular succinimides made from particular materials and having particular characteristics. More specifically, the detergent/dispersants are formed by reacting (i) triethylene tetramine or a combination of polyethylene polyamines having an average composition corresponding in general to that of triethylene tetramine, with (ii) at least one acyclic hydrocarbyl substituted succinic acylating agent in which the substituent contains an average of 50 to 90 (preferably 64 to 80) carbon atoms. In order to accomplish the objectives of this invention, it is important that the acyclic hydrocarbyl substituted succinic acylating agent have an acid number in the range of 0.9 to 1.3 (preferably in the range of 1.0 to 1.2, and most preferably 1.1). Another important feature of this invention is that the product of the reaction contain in chemically combined form from 1.5 to 2.2 (preferably from 1.9 to 2.1, most preferably 2.0) moles of (ii) per mole of (i).

The acid number of the acyclic hydrocarbyl substituted succinic acylating agent is determined in the customary way --i.e., by titration -- and is reported in terms of mg of KOH per gram of product. It is to be noted that this determination is made on the overall acylating agent with any unreacted olefin polymer (e.g., polyisobutene) present.

The acyclic hydrocarbyl substituent of the detergent/dispersant is preferably an alkyl or alkenyl group having the requisite number of carbon atoms as specified above. Alkenyl substituents derived from poly-$\alpha$-olefin homopolymers or copolymers of appropriate molecular weight (e.g., propene homopolymers, butene homopolymers, $C_3$ and $C_4$ $\alpha$-olefin copolymers, and the like) are suitable. Most preferably, the substituent is a polyisobutenyl group formed from polyisobutene having a number average molecular weight (as determined by gel permeation chromatography) in the range of 700 to 1200, preferably 900 to 1100, most preferably 940 to 1000.

Acyclic hydrocarbyl-substituted succinic acid acylating agents and methods for their preparation and use in the formation of succinimide are well known to those skilled in the art and are extensively reported in the patent literature. See for example the following U. S. Patents.

| | | |
|---|---|---|
| 3,018,247 | 3,231,587 | 3,399,141 |
| 3,018,250 | 3,272,746 | 3,401,118 |
| 3,018,291 | 3,287,271 | 3,513,093 |
| 3,172,892 | 3,311,558 | 3,576,743 |
| 3,184,474 | 3,331,776 | 3,578,422 |
| 3,185,704 | 3,341,542 | 3,658,494 |
| 3,194,812 | 3,346,354 | 3,658,495 |
| 3,194,814 | 3,347,645 | 3,912,764 |
| 3,202,678 | 3,361,673 | 4,110,349 |
| 3,215,707 | 3,373,111 | 4,234,435 |
| 3,219,666 | 3,381,022 | |

When utilizing the general procedures such as described in these patents, the important considerations insofar as the present invention is concerned, are to insure that the hydrocarbyl substituent of the acylating agent contain the requisite number of carbon atoms, that the acylating agent have the requisite acid number, that the acylating agent be reacted with the requisite polyethylene polyamine, and that the reactants be employed in proportions such that the resultant succinimide contains the requisite proportions of the chemically combined reactants, all as specified herein. When utilizing this combination of features, detergent/dispersants are formed which possess exceptional effectiveness in controlling or reducing the amount of induction system deposits formed during engine operation and which permit adequate demulsification performance.

As pointed out in the above listed patents, the acyclic hydrocarbyl-substituted succinic acylating agents include the hydrocarbyl-substituted succinic acids, the hydrocarbyl-substituted succinic anhydrides, the hydrocarbyl-substituted succinic acid halides (especially the acid fluorides and acid chlorides), and the esters of the hydrocarbyl-substituted succinic acids and lower alcohols (e.g., those containing up to 7 carbon atoms), that is, hydrocarbyl-substituted compounds which can function as carboxylic acylating agents. Of these compounds, the hydrocarbyl-substituted succinic acids and the hydrocarbyl-substituted succinic anhydrides and mixtures of such acids and anhydrides are generally preferred, the hydrocarbyl-substituted succinic anhydrides being particularly preferred.

The acylating agent used in producing the detergent/dispersants of this invention is preferably made by reacting a polyolefin of appropriate molecular weight (with or without chlorine) with maleic anhydride. However, similar carboxylic reactants can be employed such as maleic acid, fumaric acid, malic acid, tartaric acid, itaconic acid, itaconic anhydride, citraconic acid, citraconic anhydride, mesaconic acid, ethylmaleic anhydride, dimethylmaleic anhydride, ethylmaleic acid, dimethylmaleic acid and hexylmaleic acid, including the corresponding acid halides and lower aliphatic esters.

As noted above, component (i) used in the synthesis reaction for forming the detergent/dispersants of this invention is triethylene tetramine or a combination of ethylene polyamines which approximates triethylene tetramine. Ordinarily this reactant will comprise a commercially available mixture having the general overall composition approximating that of triethylene tetramine but which can contain minor amounts of branched-chain and cyclic species as well as some linear polyethylene polyamines such as diethylene triamine and tetraethylene pentamine. For best results, such mixtures should contain at least 50% and preferably at least 70% by weight of the linear polyethylene polyamines enriched in triethylene tetramine.

The reaction between components (i) and (ii) is generally conducted at temperatures of 80°C to 200°C, more preferably 140°C to 180°C, such that a succinimide is formed. These reactions may be conducted in the presence or absence of an ancillary diluent or liquid reaction medium, such as a mineral lubricating oil solvent. If the reaction is conducted in the absence of an ancillary solvent, such is usually added to the reaction product on completion of the reaction. In this way, the final product is more readily handled, stored and blended with other components. Suitable solvent oils include natural and synthetic base oils having a viscosity (ASTM D 445) of preferably 3 to 12 $mm^2/sec$ at 100°C with the primarily paraffinic mineral oils such as a 500 Solvent Neutral oil being particularly preferred. Suitable synthetic diluents include polyesters, hydrogenated or unhydrogenated poly-$\alpha$-olefins (PAO) such as hydrogenated or unhydrogenated 1-decene oligomer. Blends of mineral oil and synthetic oils are also suitable for this purpose.

As used herein, the term succinimide is meant to encompass the completed reaction product from components (i) and (ii) and is intended to encompass compounds wherein the product may have amide, amidine, and/or salt linkages in addition to the imide linkage of the type that results from the reaction of a primary amino group and an anhydride moiety.

Antioxidant.

Various compounds known for use as oxidation inhibitors can be utilized in the practice of this invention. These include phenolic antioxidants, amine antioxidants, sulfurized phenolic compounds, and organic phosphites, among others. For best results, the antioxidant should be composed predominantly or entirely of either (1) a hindered phenol antioxidant such as 2,6-di-tert-butylphenol, 4-methyl-2,6-di-tert-butylphenol, 4,4′-methylenebis(2,6-di-tert-butylphenol), and mixed methylene bridged polyalkyl phenols, or (2) an aromatic amine antioxidant such as the cycloalkyl-di-lower alkyl amines, and phenylenediamines, or a combination of one or more such phenolic antioxidants with one or more such amine antioxidants. Particularly preferred for use in the practice of this invention are combinations of tertiary butyl phenols, such as 2,6-di-tert-butylphenol, 2,4,6-tri-tert-butylphenol, o-tert-butylphenol, with N,N′-di-lower-alkyl phenylene diamines, such as N,N′-di-sec-butyl-p-phenylene diamine, and its analogs. Such combinations may contain these components in any proportions. Mixtures containing from 25 to 75 percent of (1) with 75 to 25 percent of (2) are preferred.

Demulsifier.

A wide variety of demulsifiers are available for use in the practice of this invention, including, for example, organic sulfonates, polyoxyalkylene glycols, oxyalkylated phenolic resins, and like materials. Particularly preferred are mixtures of alkylaryl sulfonates, polyoxyalkylene glycols and oxyalkylated alkylphenolic resins, such as are available commercially from Petrolite Corporation under the TOLAD trademark. One such proprietary product, identified as TOLAD 286K, is understood to be a mixture of these components dissolved in a solvent composed of alkyl benzenes. This product has been found efficacious for use in the compositions of this invention. A related product, TOLAD 286, is also suitable. In this case the product apparently contains the same kind of active ingredients dissolved in a solvent composed of heavy aromatic naphtha and isopropanol. However, other known demulsifiers can be used.

Diluent Oil.

This component of the compositions of this invention can be widely varied inasmuch as it serves the purpose of maintaining compatibility and keeping the product mixture in the liquid state of aggregation at most temperatures commonly encountered during actual service conditions. Thus use may be made of such materials as hydrocarbons, alcohols, and esters of suitable viscosity and which ensure the mutual compatibility of the other components. Preferably the diluent is a hydrocarbon, more preferably an aromatic hydrocarbon. For best results the diluent oil is most preferably an aromatic solvent with a boiling range in the region of 190-260°C and a viscosity of 1.5 to 1.9 cS at 25°C.

Corrosion Inhibitor.

Here again, a variety of materials are available for use as corrosion inhibitors in the practice of this invention. Thus, use can be made of dimer and trimer acids, such as are produced from tall oil fatty acids, oleic acid, linoleic acid, or the like. Products of this type are currently available from various commercial sources, such as, for example, the dimer and trimer acids sold under the HYSTRENE trademark by the Humko Chemical Division of Witco Chemical Corporation and under the EMPOL trademark by Emery Chemicals. Another useful type of corrosion inhibitor for use in the practice of this invention are the alkenyl succinic acid and alkenyl succinic anhydride corrosion inhibitors such as, for example, tetrapropenylsuccinic acid, tetrapropenylsuccinic anhydride, tetradecenylsuccinic acid, tetradecenylsuccinic anhydride, hexadecenylsuccinic acid, and hexadecenylsuccinic anhydride. Also useful are the half esters of alkenyl succinic acids having 8 to 24 carbon atoms in the alkenyl group with alcohols such as the polyglycols. Preferred materials are the aminosuccinic acids or derivatives thereof represented by the formula:

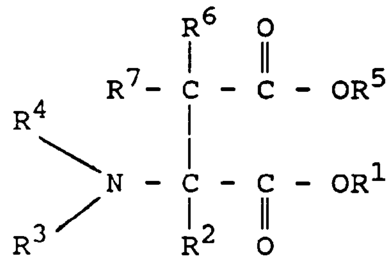

wherein each of $R^1$, $R^2$, $R^5$, $R^6$ and $R^7$ is, independently, a hydrogen atom or a hydrocarbyl group containing 1 to 30 carbon atoms, and wherein each of $R^3$ and $R^4$ is, independently, a hydrogen atom, a hydrocarbyl group containing 1 to 30 carbon atoms, or an acyl group containing from 1 to 30 carbon atoms.

The groups $R^1$, $R^2$, $R^3$, $R^4$, $R^5$, $R^6$ and $R^7$, when in the form of hydrocarbyl groups, can be, for example, alkyl, cycloalkyl or aromatic containing groups. Preferably $R^1$ and $R^5$ are the same or different straight-chain or branched-chain hydrocarbon radicals containing 1-20 carbon atoms. Most preferably, $R^1$ and $R^5$ are saturated hydrocarbon radicals containing 3-6 carbon atoms. $R^2$, either $R^3$ or $R^4$, $R^6$ and $R^7$, when in the form of hydrocarbyl groups, are preferably the same or different straight-chain or branched-chain saturated hydrocarbon radicals. Preferably a dialkyl ester of an aminosuccinic acid is used in which $R^1$ and $R^5$ are the same or different alkyl groups containing 3-6 carbon atoms, $R^2$ is a hydrogen atom, and either $R^3$ or $R^4$ is an alkyl group containing 15-20 carbon atoms or an acyl group which is derived from a saturated or unsaturated carboxylic acid containing 2-10 carbon atoms.

Most preferred is a dialkylester of an aminosuccinic acid of the above formula wherein $R^1$ and $R^5$ are isobutyl, $R^2$ is a hydrogen atom, $R^3$ is octadecyl and/or octadecenyl and $R^4$ is 3-carboxy-1-oxo-2-propenyl. In such ester $R^6$ and $R^7$ are most preferably hydrogen atoms.

The relative proportions of the various ingredients used in the additive concentrates and distillate fuels of this invention can be varied within reasonable limits. However, for best results, these compositions should contain from 5 to 35 parts by weight (preferably, from 15 to 25 parts by weight) of antioxidant, from 2 to 20 parts by weight (preferably, from 3 to 12 parts by weight) of demulsifier, and from 1 to 10 parts by weight (preferably, from 2 to 5 parts by weight) of corrosion inhibitor per each one hundred parts by weight of detergent/dispersant present in the composition. The amount of diluent oil (compatibilising oil) can be varied within considerable limits, e.g., from 5 to 150 parts by weight per hundred parts by weight of the detergent/dispersant. As noted above, the detergent/dispersant can be made in the presence of an ancillary diluent or solvent or such may be added to the detergent/dispersant after it has been produced so as to improve its handleability. Thus, in addition to the diluent oil, the concentrates and fuels may also contain from 0 to 400, preferably 250 to 400 parts, of ancillary solvent oil (dispersant/detergent carrier diluent) per 100 parts by weight of the detergent/dispersant.

The above additive compositions of this invention are preferably employed in gasolines, but are also suitable for use in middle distillate fuels, notably, diesel fuels and fuels for gas turbine engines. The nature of such fuels is so well known to those skilled in the art as to require no further comment. It will of course be understood that the base fuels may contain other commonly used ingredients such as cold starting aids, dyes, metal deactivators, octane improvers, cetane improvers, emission control additives and antioxidants.

When formulating the fuel compositions of this invention, the additives are employed in amounts sufficient to reduce or inhibit induction system deposits in an internal combustion engine. Generally speaking, the additives will be employed in amounts such that the fuel contains on a weight basis from 60 to 250, and preferably from 130 to 190 parts of the detergent/dispersant per million parts of fuel. The other components which are preferably used in conjunction with the detergent/dispersant can be blended into the fuel individually or in various sub-combinations. However, it is definitely preferable to blend all of the components concurrently using an additive concentrate of this invention as this takes advantage of the mutual compatibility afforded by the combination of ingredients when in the form of an additive concentrate.

The following Examples in which all parts are by weight illustrate, but are not intended to limit, this invention.

Example 1

A fuel additive concentrate is prepared from the following ingredients:

(a) 135 parts of a detergent/dispersant formed by reacting polyisobutenylsuccinic anhydride having an acid number of 1.1 (made by reaction of maleic anhydride and polyisobutene having a number average molecular weight of 950) with a commercial mixture approximating triethylene tetramine, in a mole ratio of 2:1

respectively. This component also includes 500 parts of 500 Solvent Neutral mineral oil (serving as a carrier for the detergent/dispersant).

(b) 30 parts of a mixture of 15 parts of N,N'-di-sec-butyl-p-phenylenediamine and 15 parts of a tertiary butylated phenol antioxidant mixture containing a minimum of 75 percent of 2,6-di-tert-butylphenol, 10-15 percent of 2,4,6-tri-tert-butylphenol, and 15-10 percent of 2-tert-butylphenol.

(c) 10 parts of a demulsifier mixture composed of alkylaryl sulfonates, polyoxyalkylene glycols and oxyalkylated alkylphenolic resins in alkylbenzenes (TOLAD 286K).

(d) 120 parts of an aromatic solvent with a boiling range of 196-256°C and a viscosity of 1.7 cS at 25°C.

(e) 5 parts of aspartic acid, N-(3-carboxy-1-oxo-2-propenyl)-N-octadecyl-bis(2-methylpropyl) ester.

This concentrate is blended with gasolines and with diesel fuels at concentrations of 400, 800, 1200 and 2000 ppm.

Example 2

Example 1 is repeated except that component (e) is omitted.

Example 3

Example 1 is repeated using each of the components set forth therein except that 150 parts of component (a) and 105 parts of component (d) are used.

Example 4

Example 1 is repeated using as component (a) 135 parts of a detergent/dispersant formed by reacting polyisobutenylsuccinic anhydride (made by reaction of maleic anhydride and polyisobutene having a number average molecular weight of 750) and an acid number of 1.2 with triethylene tetramine in a mole ratio of 1.8:1 respectively. No ancillary diluent is employed in component (a).

Example 5

Example 1 is repeated using as component (a) 135 parts of a detergent/dispersant formed by reacting polyisobutenylsuccinic anhydride with an acid number of 1.0 (made by reaction of maleic anhydride and polyisobutene having a number average molecular weight of 1200) with triethylene tetramine in a mole ratio of 2.2:1 respectively. This detergent/dispersant is in admixture with 400 parts of 500 Solvent Neutral mineral oil as a carrier oil.

Example 6

Example 1 is repeated with the following changes: Component (a) is 170 parts of the detergent/dispersant admixed with 520 parts of 500 Solvent Neutral Oil, the acid number of the polyisobutenylsuccinic anhydride used in making the detergent dispersant is 0.9, and 65 parts of component (d) are used.

The effectiveness of the compositions of this invention in reducing induction system deposits was demonstrated in a series of standard four-cylinder engine tests using a test gasoline containing 800 ppm of an additive concentrate as in Example 1. In one set of tests -- the Volkswagen Waterboxer Valve Sticking Test -- the test engine was operated under a predetermined series of operating conditions involving different speeds and loads. Measurements were made of compression pressures of the individual cylinders of the engine in order to determine whether any valve sticking occurred during the tests. It was determined that no valve sticking occurred.

In another test using a Daimler-Benz 102E 2.3 liter engine, the test gasoline, which contained 800 ppm of an additive concentrate as in Example 6, was subjected to a sixty hour test procedure prescribed by Daimler-Benz AG in order to measure intake system cleanliness. Measurements were made both of the inlet valve deposits and of the tulip deposits. In addition, each inlet valve was rated visually for cleanliness and type of deposits formed thereon. It was found that the total deposits formed on the valve and the tulip surfaces amounted to 299 mg, whereas the same fuel which did not contain the additive concentrate of this invention yielded 959 mg of such deposits under the same test conditions. The tulip deposits resulting from use of the fuel of this invention totaled 208 mg whereas the same base fuel devoid of the additive concentrate of this invention gave 935 mg of tulip deposits. The visual ratings were, in general, very good. In another test in which an additive concentrate as in Example 1 was used in place of an additive concentrate of Example 6, the results obtained

were: total deposits formed on the valve and the tulip surfaces, 655 mg; tulip deposits, 554 mg.

The ability of the compositions of this invention to shed water was illustrated by means of demulsification tests conducted using the ASTM D 1094 demulsification test procedure. The base gasoline fuel used in these tests was a premium leaded gasoline. This fuel was tested without any additional additives (control), and in another case with a content of 800 ppm of the additive concentrate of Example 1. For comparative purposes another portion of the base fuel containing 800 ppm of an additive concentrate not of this invention was tested. This additive concentrate was the same as in Example 1 except that:

1) The detergent/dispersant was made from polyisobutenylsuccinic anhydride having an acid number of 0.9 and tetraethylene pentamine in a mole ratio of 1.8:1;
2) Component (a) consisted of 225 parts of such detergent/dispersant -- no carrier diluent therefor was employed;
3) Components (b) and (e) were not present;
4) Component (c) was present at 40 parts;
5) Component (d) was present at 375 parts.

The results of these tests are summarized in the following table.

| Additive Concentrate | Phase Separation (Emulsion Layer) $mm^3$ | |
|---|---|---|
| | pH 7 Buffer | pH 9 Buffer |
| None | 0 | 0 |
| Example 1 | 0.5 | 0 |
| Comparative | 22 | 20 |

## Claims

1. A detergent/dispersant prepared by a process which comprises reacting (i) triethylene tetramine or a combination of ethylene polyamines which approximate triethylene tetramine with (ii) at least one acyclic hydrocarbyl substituted succinic acylating agent characterized in that the substituent contains an average of 50 to 90 carbon atoms, and in that such acylating agent has an acid number in the range of 0.9 to 1.3, said detergent/dispersant containing in its molecular structure in chemically combined form an average of from 1.5 to 2.2 moles of (ii) per mole of (i).

2. A composition as claimed in Claim 1 wherein the hydrocarbyl substituent of the succinic acylating agent is a polyisobutenyl group.

3. A composition as claimed in either of Claims 1 and 2 wherein the hydrocarbyl substituent contains an average of 64 to 80 carbon atoms, wherein the acid number is in the range of 1.0 to 1.2, and wherein said molecular structure contains in chemically combined form an average of from 1.9 to 2.1 moles of (ii) per mole of (i).

4. A distillate fuel containing a minor but effective amount of (a) a detergent/dispersant as claimed in any one of Claims 1 to 3, and optionally, (b) an antioxidant, or (c) a demulsifier, or (d) a diluent oil, or (e) a corrosion inhibitor, or any combination of any two or any three or all four of components (b), (c), (d) and (e).

5. An additive concentrate which comprises, in combination, (a) a detergent/dispersant as claimed in any one of Claims 1 to 3, and (b) an antioxidant, or (c) a demulsifier, or (d) a diluent oil, or (e) a corrosion inhibitor, or any combination of any two or any three or all four of components (b), (c), (d) and (e).

6. A composition as claimed in either of Claims 4 and 5 wherein the composition includes component (b), and wherein component (b) is composed predominantly or entirely of at least one hindered phenol antioxidant or at least one phenylene diamine antioxidant, or a combination of at least one hindered phenol

antioxidant and at least one phenylene diamine antioxidant.

7. A composition as claimed in any of Claims 4 to 6 wherein the composition includes component (c), and wherein component (c) comprises one or more alkylaryl sulfonates or one or more polyalkylene glycols or one or more oxyalkylated alkylphenolic resins, or any mixtures of such materials.

8. A composition as claimed in any one of Claims 4 to 7 wherein the composition includes component (d).

9. A composition as claimed in any one of Claims 4 to 8 wherein the composition includes component (e), and wherein component (e) is composed predominantly or entirely of at least one compound represented by the formula

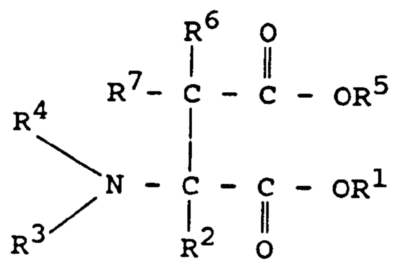

wherein each of $R^1$, $R^2$, $R^5$, $R^6$ and $R^7$ is, independently, a hydrogen atom or a hydrocarbyl group containing 1 to 30 carbon atoms, and wherein each of $R^3$ and $R^4$ is, independently, a hydrogen atom, a hydrocarbyl group containing 1 to 30 carbon atoms, or an acyl group containing from 1 to 30 carbon atoms.

10. Gasoline or a gasoline additive concentrate characterized by containing, in combination, (a) a detergent/dispersant as claimed in any one of Claims 1 to 3, and (b) an antioxidant composed predominantly or entirely of at least one hindered phenol antioxidant or at least one phenylene diamine antioxidant, or a combination of at least one hindered phenol antioxidant and at least one phenylene diamine antioxidant, and (c) a demulsifier composed predominantly or entirely of one or more alkylaryl sulfonates or one or more polyalkylene glycols or one or more oxyalkylated alkylphenolic resins, or any mixtures of such materials, and (d) a diluent oil, and (e) a corrosion inhibitor composed predominantly or entirely of aspartic acid, N-(3-carboxy-1-oxo-2-propenyl)-N-octadecyl-bis(2-methylpropyl) ester, said components being present in proportions such that per each 100 parts by weight of component (a), there are 15 to 25 parts by weight of component (b), 3 to 12 parts by weight of component (c), and 2 to 5 parts by weight of component (e).

## Patentansprüche

1. Ein Detergens/Dispergens hergestellt durch ein Verfahren, bei dem man (i) Triethylentetramin oder eine mit Triethylentetramin vergleichbare Kombination von Ethylenpolyaminen mit (ii) mindestens einem acyclischen hydrocarbylsubstituierten succinacylierenden Mittel umsetzt, dadurch gekennzeichnet, daß der Substituent im Durchschnitt 50 bis 90 Kohlenstoffatome aufweist, und dadurch, daß das Acylierungsmittel eine Säurezahl im Bereich von 0,9 bis 1,3 besitzt, wobei das Detergens/Dispergens in seiner molekularen Struktur in chemisch kombinierter Form durchschnittlich 1,5 bis 2,2 Mol von (ii) pro Mol von (i) enthält.

2. Zusammensetzung nach Anspruch 1, in der der Hydrocarbylsubstituent des succinacylierenden Mittels eine Polyisobutenylgruppe ist.

3. Zusammensetzung nach einem der Ansprüche 1 oder 2, in der der Hydrocarbylsubstituent im Durchschnitt 64 bis 80 Kohlenstoffatome aufweist, die Säurezahl im Bereich von 1,0 bis 1,2 liegt und worin die molekulare Struktur in chemisch kombinierter Form durchschnittlich 1,9 bis 2,1 Mol von (ii) pro Mol von (i) enthält.

4. Destillattreibstoff enthaltend eine kleinere jedoch wirksame Menge von (a) einem Detergens/Dispergens nach einem der Ansprüche 1 bis 3 und gegebenenfalls (b) ein Antioxidans oder (c) ein Demulgiermittel oder (d) ein Verdünnungsöl oder (e) einen Korrosionsinhibitor oder eine beliebige Kombination von beliebigen zwei oder beliebigen drei oder allen vier Komponenten (b), (c), (d) und (e).

5. Additivkonzentrat, das in Kombination (a) ein Detergens/Dispergens nach einem der Ansprüche 1 bis 3 und (b) ein Antioxidans oder (c) ein Demulgiermittel oder (d) ein Verdünnungsöl oder (e) einen Korrosionsinhibitor oder eine beliebige Kombination von beliebigen zwei oder beliebigen drei oder allen vier der Komponenten (b), (c), (d) und (e) enthält.

6. Zusammensetzung nach einem der Ansprüche 4 oder 5, in der die Zusammensetzung die Komponente (b) umfaßt und worin die Komponente (b) überwiegend oder vollständig aus mindestens einem gehinderten Phenol-Antioxidans oder mindestens einem Phenylendiamin-Antioxidans oder aus einer Kombination von mindestens einem gehinderten Phenol-Antioxidans und mindestens einem Phenylendiamin-Antioxidans zusammengesetzt ist.

7. Zusammensetzung nach einem der Ansprüche 4 bis 6, worin die Zusammensetzung die Kompenente (c) einschließt und worin die Komponente (c) ein oder mehrere Alkylarylsulfonate oder ein oder mehrere Polyalkylenglykole oder ein oder mehrere oxylalkylierte alkylphenolische Harze oder eine Mischung von solchen Materialen enthält.

8. Zusammensetzung nach einem der Ansprüche 4 bis 7, in der die Zusammensetzung die Komponente (d) einschließt.

9. Zusammensetzung nach einem der Ansprüche 4 bis 8, worin die Zusammensetzung die Komponente (e) einschließt, und worin die Komponente (e) überwiegend oder vollständig aus einer Verbindung entsprechend der Formel

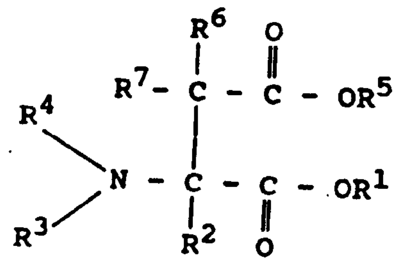

zusammengesetzt ist, in der jeder Rest $R^1$, $R^2$, $R^5$, $R^6$ und $R^7$ unabhängig ein Wasserstoffatom oder eine Hydrocarbylgruppe mit 1 bis 30 Kohlenstoffatomen bedeutet, und worin jeder Rest $R^3$ und $R^4$ unabhängig ein Wasserstoffatom, eine Hydrocarbylgruppe mit 1 bis 30 Kohlenstoffatomen oder eine Acylgruppe mit 1 bis 30 Kohlenstoffatomen bedeutet.

10. Benzin oder ein Benzinadditivkonzentrat dadurch gekennzeichnet, daß es in Kombination, (a) ein Detergens/Dispergens nach einem der Ansprüche 1 bis 3 und (b,) ein Antioxidans, zusammengesetzt überwiegend oder vollständig aus mindestens einem gehinderten Phenol-Antioxidans oder mindestens einem Phenylendiamin-Antioxidans oder eine Kombination aus mindestens einem gehinderten Phenol-Antioxidans oder mindestens einem Phenylendiamin-Antioxidans und (c) ein Demulgiermittel zusammengesetzt überwiegend oder vollständig aus einem oder mehreren Alkylarylsulfonaten oder einem oder mehreren Polyalkylenglykolen oder einem oder mehreren oxyalkylierten alkylphenolischen Harzen oder jedwede Mischung von solchen Materialien und (d) ein Verdünnungsöl und (e) einen Korrosioninhibitor zusammengesetzt überwiegend oder vollständig aus Asparaginsäure, N-(3-Carboxy-1-oxo-2-propenyl)-N-octadecyl- bis (2-methylpropyl)-Ester, wobei die Komponenten in Anteilen vorhanden sind, so daß pro jeweils 100 Gewichtsteilen der Komponente (a) 15 bis 25 Gewichtsteile der Komponente (b), 3 bis 12 Gewichtsteile der Komponente (c) und 2 bis 5 Gewichtsteile der Komponente (e) vorhanden sind, enthält.

## Revendications

1. Détergent/dispersant préparé par un procédé qui comprend la réaction (i) de triéthylènetétramine ou d'un mélange d'éthylènepolyamines,correspondant approximativement à la triéthylènetétramine, avec (ii) au moins un agent succinique d'acylation à substituant hydrocarbyle acyclique, caractérisé en ce que le substituant contient un nombre moyen de 50 à 90 atomes de carbone, et en ce que cet agent d'acylation possède un indice d'acide compris dans l'intervalle de 0,9 à 1,3, ledit détergent/dispersant contenant dans sa structure moléculaire, sous une forme chimiquement combinée, un nombre moyen de 1,5 à 2,2 moles de constituant (ii) par mole de constituant (i).

2. Composition suivant la revendication 1, dans laquelle le substituant hydrocarbyle de l'agent succinique d'acylation est un groupe polyisobutényle.

3. Composition suivant chacune des revendications 1 et 2, dans laquelle le substituant hydrocarbyle contient un nombre moyen de 64 à 80 atomes de carbone, l'indice d'acide est compris dans l'intervalle de 1,0 à 1,2, et la structure moléculaire contient, sous une forme chimiquement combinée, un nombre moyen de 1,9 à 2,1 moles de constituant (ii) par mole de constituant (i).

4. Combustible distillé contenant une quantité faible mais efficace (a) d'un détergent/dispersant suivant l'une quelconque des revendications 1 à 3, et facultativement, (b) d'un anti-oxydant, ou (c) d'un désémulsionnant, ou (d) d'une huile diluante, ou (e) d'un inhibiteur de corrosion, ou bien de n'importe quelle association de deux quelconques ou trois quelconques ou de l'ensemble des quatre constituants (b), (c), (d) et (e).

5. Concentré d'additifs qui comprend, en association, (a) un détergent/dispersant suivant l'une quelconque des revendications 1 à 3, et (b) un anti-oxydant, ou (c) un désémulsionnant, ou (d) une huile diluante, ou (e) un inhibiteur de corrosion, ou bien n'importe quelle association de deux quelconques ou trois quelconques ou bien de l'ensemble des quatre constituants (b), (c), (d) et (e).

6. Composition suivant chacune des revendications 4 et 5, qui comprend le constituant (b) et dans laquelle le constituant (b) est constitué principalement ou totalement d'au moins un anti-oxydant phénolique à encombrement stérique ou d'au moins un anti-oxydant du type phénylènediamine, ou bien d'une association d'au moins un anti-oxydant phénolique à encombrement stérique et d'au moins un anti-oxydant du type phénylènediamine.

7. Composition suivant l'une quelconque des revendications 4 à 6, qui renferme le constituant (c) et dans laquelle le constituant (c) comprend un ou plusieurs alkylarylsulfonates, ou un ou plusieurs polyalkylèneglycols, ou une ou plusieurs résines alkylphénoliques oxyalkylées, ou bien n'importe quels mélanges de ces substances.

8. Composition suivant l'une quelconque des revendications 4 à 7, qui renferme le constituant (d).

9. Composition suivant l'une quelconque des revendications 4 à 8, qui renferme le constituant (e) et dans laquelle le constituant (e) est constitué principalement ou totalement d'au moins un composé représenté par la formule

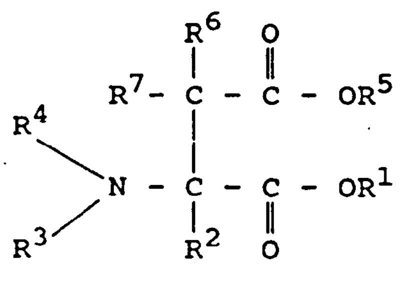

dans laquelle chacun des groupes $R^1$, $R^2$, $R^5$, $R^6$ et $R^7$ représente, indépendamment, un atome d'hydrogène ou un groupe hydrocarbyle contenant 1 à 30 atomes de carbone, et dans laquelle chacun des groupes $R^3$ et $R^4$ représente, indépendamment, un atome d'hydrogène, un groupe hydrocarbyle contenant 1 à 30 atomes de carbone, ou un groupe acyle contenant 1 à 30 atomes de carbone.

10. Essence ou concentré d'additifs pour essence caractérisé par la présence, en association, (a) d'un détergent/dispersant suivant l'une quelconque des revendications 1 à 3, et (b) d'un anti-oxydant constitué principalement ou totalement d'au moins un anti-oxydant phénolique à encombrement stérique ou d'au moins un anti-oxydant du type phénylènediamine, ou bien d'une association d'au moins un anti-oxydant

phénolique à encombrement stérique et d'au moins un anti-oxydant du type phénylènediamine, et (c) d'un désémulsionnant constitué principalement ou totalement d'un ou plusieurs alkylarylsulfonates, ou d'un ou plusieurs polyalkylèneglycols, ou d'une ou plusieurs résines alkylphénoliques oxyalkylées, ou bien de n'importe quels mélanges de ces substances, et (d) d'une huile diluante, et (e) d'un inhibiteur de corrosion constitué principalement ou totalement d'ester de N-(3-carboxy-1-oxo-2-propényl)-N-octadécylbis(2-méthylpropyle) d'acide aspartique, lesdits constituants étant présents en des proportions telles que, pour chaque quantité de 100 parties en poids de constituant (a), il existe 15 à 25 parties en poids de constituant (b), 3 à 12 parties en poids de constituant (c) et 2 à 5 parties en poids de constituant (e).